# EUROPEAN PATENT APPLICATION

(11) **EP 4 553 164 A2**
(43) Date of publication of application: **14.05.2025**
(21) Application number: 23834957.5
(22) Date of filing: 10.07.2023
(51) Int. Cl.: C12N 15/86, C12N 15/50, A61K 9/127, A61K 39/215, A61K 47/28, A61P 31/14

(54) **MODIFIED SELF-REPLICATING MRNA**

(30) Priority: 08.07.2022 CN 202210805852
(71) Applicant: IMMORNA (HANGZHOU) BIOTECHNOLOGY CO., LTD., Hangzhou, Zhejiang 311215 (CN)
(72) Inventor: WANG, Zihao, Hangzhou, Zhejiang 311215 (CN); REN, Xiaoguang, Hangzhou, Zhejiang 311215 (CN); TANG, Zhewei, Hangzhou, Zhejiang 311215 (CN); CHEN, Yanni, Hangzhou, Zhejiang 311215 (CN)
(74) Representative: Brann AB
(86) International application number: PCT/CN2023/106610
(87) International publication number: WO 2024/008192

(57) **Abstract**

The present invention belongs to the field of nucleotides and mRNA, and specifically relates to modified self-replicating mRNA. A polynucleotide fragment thereof comprises an integrated target fragment and one or more non-structural replicase domains from an α virus, the integrated target fragment comprising a functional nucleotide analogue; and the functional nucleotide analogue comprises: at least one among pseudouridine, N1-methylpseudouridine, 5-hydroxymethoxycytidine, and N⁶-methyladenosine.

## Description

### CROSS-REFERENCE TO RELATED APPLICATIONS

This application claims the priority of patent application No. CN 2022108058522, filed on July 8, 2022, which is incorporated herein by reference in its entirety.

### TECHNICAL FIELD

The present disclosure belongs to the field of nucleotides and mRNAs, and specifically relates to modified self-replicating mRNA.

### BACKGROUND

Traditional inactivated vaccines and recombinant protein vaccines are produced with long cycles and complex processes, and thus are unsuitable for mass vaccination in response to sudden large-scale epidemics. mRNA is a rapid-response vaccine development platform for explosive outbreaks. In 2020, nucleotide-modified mRNA (i.e. messenger ribonucleic acid) vaccines developed by Moderna and BioNTech were marketed as mRNA-1273 and Tozinameran, respectively, to combat the COVID-19 pandemic. These vaccines, aimed at preventing COVID-19 through vaccination, proved the safety and effectiveness of mRNA vaccines.

The mRNA vaccine is synthesized through an *in vitro* enzymatic transcription reaction using linearized plasmid DNA as a template. This synthesis strategy circumvents issues related to live cell culture and production methods, safety concerns, and complex manufacturing processes. The mRNA vaccine platform is characterized by its safety, effectiveness, short production cycle, and simplicity in processing, and thus is particularly suitable for addressing explosive epidemics.

A self-replicating mRNA vaccine demonstrates strong immunogenicity in animal experiments and can replicate its own sequence using itself as a template. Consequently, the self-replicating mRNA vaccine requires a lower inoculation dose compared to conventional mRNA vaccines. Additionally, the adjuvant effect created by the immune response during self-replication can induce a stronger immune response, thereby further enhancing both humoral and cellular immune responses.

However, further studies are required for the nucleotide-modified self-replicating mRNA.

### SUMMARY OF THE INVENTION

A first objective of the present disclosure is to provide a modified self-replicating mRNA, where a polynucleotide fragment of the modified self-replicating mRNA includes one or more non-structural replicase domains from an α virus and an integrated target fragment, where the polynucleotide fragment includes a functional nucleotide analog; and
the functional nucleotide analog includes at least one of pseudouridine, N1-methylpseudouridine, 5-hydroxymethoxycytidine, and N⁶-methyladenosine.

A second objective of the present disclosure is to provide a vaccine composition including the modified self-replicating mRNA.

A third objective of the present disclosure is to provide a kit including the vaccine composition and optionally a container for administering the vaccine composition.

A fourth objective of the present disclosure is to provide a method for modifying the following properties of mRNA after inoculation into an animal:
a) improvement of immunogenicity of the mRNA; and/or
b) improvement of the mRNA's ability to evade immune surveillance,
wherein the method includes substituting an unmodified nucleotide in the mRNA with a corresponding functional nucleotide analog.

Studies have shown that the modified self-replicating mRNA exhibits a better expression effect than self-replicating mRNA without nucleotide modification. Additionally, it has been found that the modified self-replicating mRNA can evade immune surveillance and induce relatively weaker transcription of IFN-B1 and/or RIG-1 in cell experiments. Consequently, this allows for prolonged expression and the induction of an immune response.

### BRIEF DESCRIPTION OF THE DRAWINGS

To more clearly illustrate the technical solutions in the specific embodiments or prior art of the present desclosure, the drawings needed for the description are briefly introduced below. Apparently, the drawings described below are some embodiments of the present disclosure. For those of ordinary skill in the art, other drawings can also be obtained based on these drawings without creative efforts.
FIG. 1 is a structural map of mRNA-RBD plasmid according to an example of the present disclosure, which contains a kanamycin resistance gene (Kan), a T7 promoter element, a nsp gene, a RBD gene, and a polyA element.
FIG. 2 is a diagram of electrophoresis results of a self-replicating mRNA-RBD (denoted as SAM-CD5-RBD in the figure) after *in vitro* transcription according to an example of the present disclosure, where Ψ-UTP, 1mΨ-UTP and 5m-CTP represent pseudouridine, N1-methylpseudouridine and 5-hydroxymethoxycytidine respectively, 0%, 10%, 20%, ..., 90%, and 100% represent the proportions of pseudouridine, N1-methylpseudouridine and 5-hydroxymethoxycytidine modifications during *in vitro* transcription of the self-replicating mRNA-RBD, and M represents RNA molecular weight standard (RNA ladder).

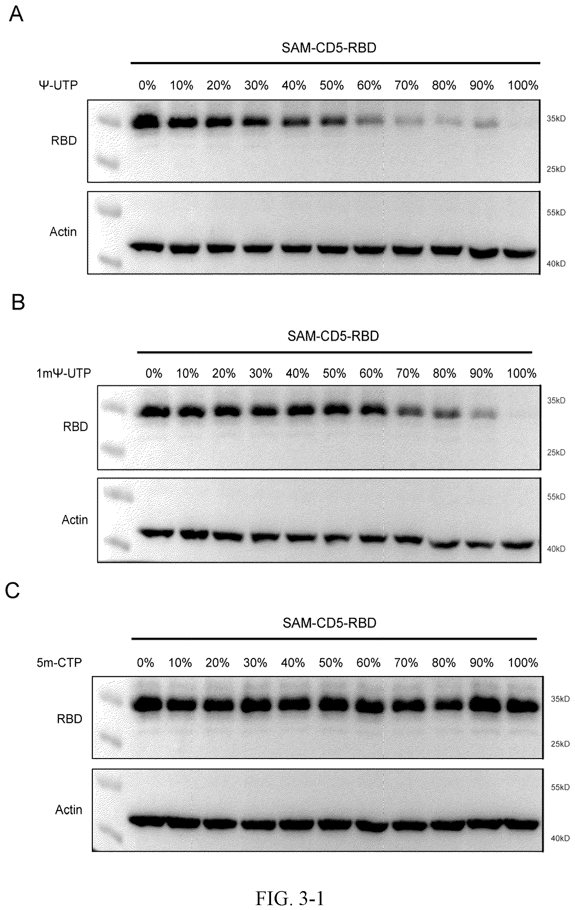

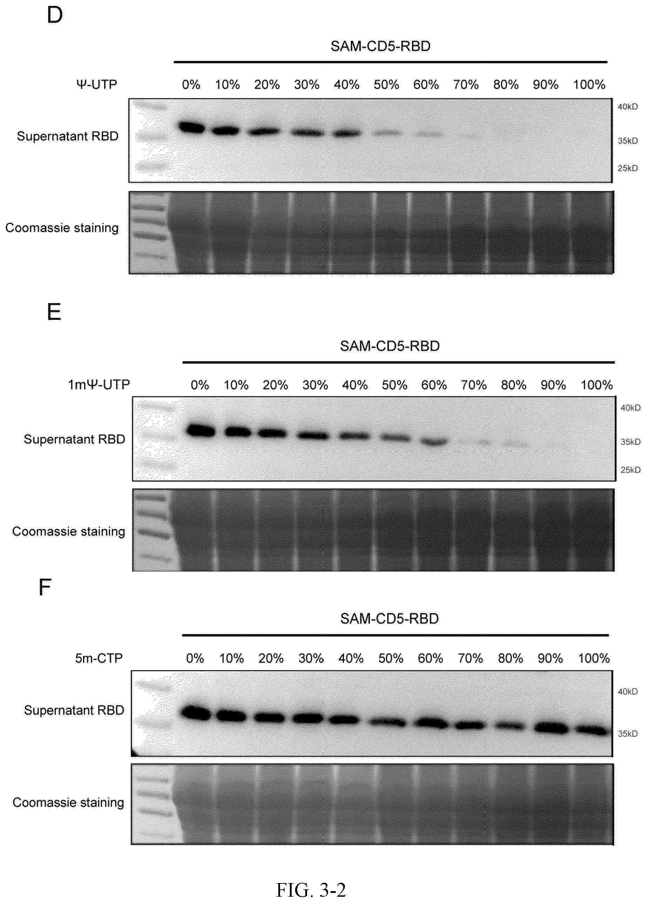

FIG. 3 is a diagram comparing the expression of RBD proteins detected by western blot after BHK cells are transfected *in vitro* with self-replicating mRNA-RBD (denoted as SAM-CD5-RBD in the figure) with modified and conventional nucleotides according to an example, where A to C represent the RBD expression results of self-replicating mRNA-RBD with different proportions of modified nucleotides of pseudouridine, N1-methylpseudouridine and 5-hydroxymethoxycytidine in cell lysates, and D to F represent RBD expression results of self-replicating mRNA-RBD with different proportions of modified nucleotides of pseudouridine, N1-methylpseudouridine and 5-hydroxymethoxycytidine in cell supernatants.

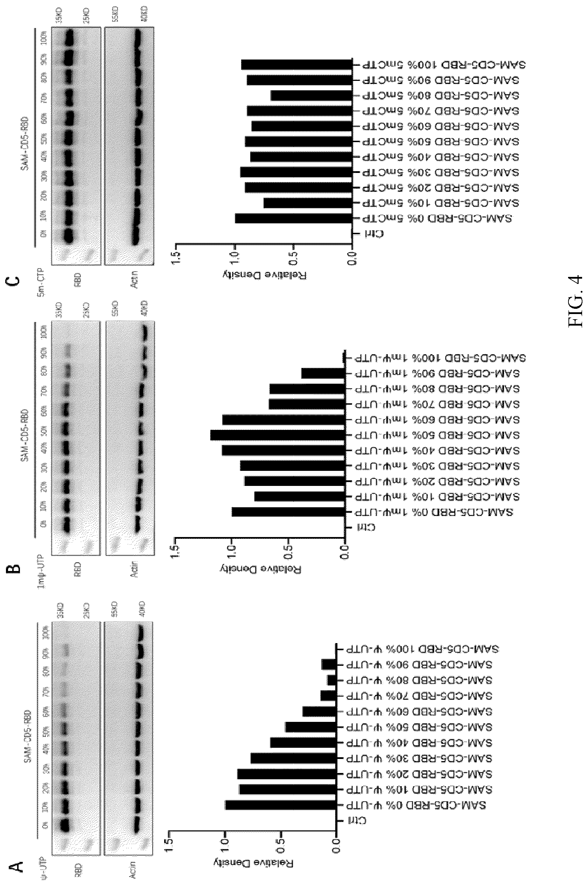

FIG. 4 is a diagram comparing the expression of RBD proteins detected by western blot after BHK cells are transfected *in vitro* with self-replicating mRNA-RBD (denoted as SAM-CD5-RBD in the figure) with modified nucleotides and conventional nucleotides, according to an example. The expression of RBD proteins is quantitatively analyzed by using Actin as internal reference. The upper parts of A to C represent RBD expression results of self-replicating mRNA-RBD with different proportions of modified nucleotides of pseudouridine, N1-methylpseudouridine and 5-hydroxymethoxycytidine in cell lysates, and the lower parts of A to C correspond to the quantitative analysis of RBD expression.
FIG. 5 is a diagram comparing the expression of innate immunomodulator IFN-B1 gene, detected by qPCR, after Hela cells are transfected *in vitro* with self-replicating mRNA-RBD (denoted as SAM-CD5-RBD in the figure) with modified nucleotides and conventional nucleotides, according to an example.
FIG. 6 is a diagram comparing the expression of the IFN-B1 gene in mouse spleen cells, detected by qPCR, using self-replicating mRNA-RBD (denoted as SAM-CD5-RBD in the figure) with modified nucleotides and conventional nucleotides, according to an example.

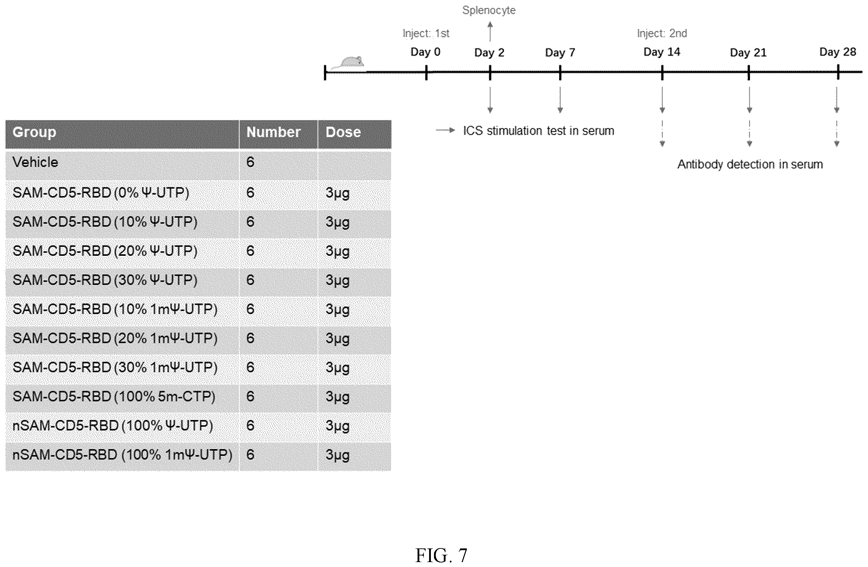

FIG. 7 shows the design solution of an animal experiment according to an example. Six- to eight-week-old BALB/c female mice were divided into groups after 3 days of adaptive feeding, with 6 mice in each group. One group served as the control group and was injected with a buffer solution. The other ten groups were respectively injected with various self-replicating mRNA-RBD-LNP formulations as follows: self-replicating mRNA-RBD-LNP with conventional nucleotides (denoted as SAM-CD5-RBD (0% ψ-UTP)), 10%, 20%, and 30% pseudouridine-modified self-replicating mRNA-RBD-LNP (denoted as SAM-CD5-RBD (10%, 20%, 30% ψ-UTP)), 10%, 20%, and 30% N1-methylpseudouridine-modified self-replicating mRNA-RBD-LNP (denoted as SAM-CD5-RBD (10%, 20%, 30% 1mψ-UTP)), 100% 5-methylcytidine-modified self-replicating mRNA-RBD-LNP (denoted as SAM-CD5-RBD (100% 5m-CTP)), 100% pseudouridine-modified non-self-replicating mRNA-RBD-LNP (denoted as nSAM-CD5-RBD (100% ψ-UTP)), and 100% N1-methylpseudouridine-modified non-self-replicating mRNA-RBD-LNP (denoted as nSAM-CD5-RBD (100% 1mψ-UTP)). Each mouse was injected with 3 µg of mRNA-RBD-LNP in the spine on day 0. The spleens of three mice in each group were collected on day 2 to detect IFN-B1 expression. For the remaining three mice in each group, serum was drawn on days 7 and 14, followed by a second injection of 3 µg mRNA-RBD-LNP in the spine. Serum was then collected on days 21 and 28 to detect antibody titers against RBD.
FIG. 8 shows the titer detection of serum antibodies in the animal experiment according to an example, where the antibody specific to RBD in the serum is detected by ELISA. The ordinate shows the value of the logarithm of the antibody titer, and the abscissa represents the corresponding samples.
FIG. 9 shows the effect of m⁶A modification on SAM-RNA expression according to an example.
FIG. 10 shows the results of RBD expression detected by Western blot (WB) according to an example.
FIG. 11 shows the evaluation of the effect of m⁶A modification on the expression of the IFN-B1 gene according to an example, where it can be seen that m⁶A modification can effectively reduce IFN-B 1 expression.

### DETAILED DESCRIPTION

Detailed references to embodiments of the present disclosure are provided below, with one or more examples described. Each example is provided to illustrate rather than limit the present disclosure. It is apparent to those skilled in the art that various modifications and changes can be made to the present disclosure without departing from its scope or spirit. For example, features illustrated or described as part of one embodiment can be used in another embodiment to create yet another embodiment.

Unless otherwise specified, all terms (including technical and scientific terms) used to disclose the present disclosure have the same meanings as commonly understood by persons of ordinary skill in the relevant art. Further guidance and subsequent definitions are used to better understand the teachings of the present disclosure. The terms used in this description are solely for the purpose of describing specific embodiments and are not intended to limit the present disclosure.

The selection range of terms such as "and/or", "or/and", and "and/or" includes any one of two or more related listed items, as well as any combination of any one or all of the related listed items. These combinations include any two related listed items, any additional related listed items, or all related listed items. It should be noted that when at least three items are conjoined by "and/or", it is understood that the technical solution undoubtedly includes both the "logical and" and the "logical or" connections. For example, "A and/or B" includes A, B, and A+B. Similarly, "A, and/or B, and/or C, and/or D" includes any one of A, B, C, or D (i.e., connected by "logical or"), as well as any combination of A, B, C, and D, including any two or three of them, and all four of them (i.e., connected by "logical and").

The terms "comprise", "comprising", "comprises", "include", "includes", "including", "contain", "contains", and "containing" as used in the present disclosure are synonymous, and are inclusive or open-ended, and do not exclude additional uncited members, elements or method steps.

A value range in the present disclosure that is defined by endpoints includes all values, fractions, and the cited endpoints subsumed within the range.

A concentration value involved in the present disclosure may fluctuate within a specific range. For example, the concentration value may fluctuate within a corresponding precision range. For instance, a precision range of 2% allows fluctuation within ±0.1%. A larger fluctuation is also allowed for a larger value or a value that does not require fine control. For example, a precision range of 100 mM can allow fluctuation within ±1%, ±2%, ±5%, and so on. Molecular weight is allowed to fluctuate by ±10%.

In the present disclosure, descriptions such as "multiple" and "various" refer to two or more, unless otherwise specified.

In the present disclosure, technical features in open-ended descriptions include closed-ended technical solutions consisting of the listed features, as well as open-ended technical solutions including the listed features.

In the present disclosure, terms like "preferred", "preferably", and "preferable" are only intended to describe embodiments or examples with better effects, and should not be construed as limitations on the protection scope of the present disclosure. In the present disclosure, "optionally" and "optional" refer to "dispensable" or any one of two parallel solutions of "with" and "without". If "optional" appears multiple times in a technical solution, the "optional" at each location is independent unless a specific explanation is provided and there are contradictions or mutual constraints.

As used herein, the term "functional nucleotide analog" refers to modified forms of classic nucleotides A, G, C, U, or T. These forms (a) retain the base-pairing characteristics of the corresponding classic nucleotides and (b) contain at least one chemical modification of (i) nucleobase, (ii) glycosyl group, (iii) phosphate group, or (iv) any combination of (i) to (iii) of the corresponding natural nucleotides. As used herein, base pairing covers not only classical Watson-Crick base pairs of adenine-thymine, adenine-uracil, or guanine-cytosine but also base pairs formed between classic nucleotides and functional nucleotide analogs or between two functional nucleotide analogs. In these cases, the arrangement of hydrogen bond donors and acceptors allows the formation of hydrogen bonds between modified nucleobases and classic nucleobases or between two complementary modified nucleobase structures. For example, a functional analog of guanosine (G) retains the ability to form a base pair with cytosine (C) or a functional analog of cytosine. An example of such non-classical base pairing is the base pairing between modified nucleotide inosine and adenine, cytosine, or uracil. As described herein, functional nucleotide analogs can be naturally occurring or artificial. Therefore, a nucleic acid molecule containing functional nucleotide analogs can have at least one modified nucleobase, glycosyl group, and/or internucleoside linkage. Exemplary chemical modifications to the nucleobases, glycosyl groups, or internucleoside linkages of nucleic acid molecules are provided herein. Examples of functional nucleotide analogs include one, two or more of 4-acetylcytidine, 5-(carboxyhydroxymethyl)uridine, dihydrouridine, 2'-O-methylpseudouridine, β,D-galactose Q nucleoside, 2'-O-methylguanosine, inosine, N⁶-isopentenyladenosine, 1-methyladenosine, 1-methylpseudouridine, 1-methylinosine, 2'2-dimethyladenosine, 2-methyladenosine, 2-methylguanosine, 5-methyluridine, 3-methylcytidine, 5-methylcytidine, N⁶-methyladenosine, 7-methylguanosine, 5-methylaminomethyluridine, 5-carboxymethylaminomethyluridine, 5-carboxymethylaminomethyl-2-thiouridine, β,D-mannose Q nucleoside, 5-methoxycarbonylmethyl-2-thiouridine, 5-methoxycarbonyl-methyluridine, 5-methoxyuridine, 2-thiomethyl-N⁶-isopentenyladenosine, N-((9-β-D-ribofuranosyl-2-thiomethylpurin-6-yl)carbamoyl)threonine, N-((9-β-D-ribofuranosyl-purin-6-yl)N-methylcarbamoyl)threonine, uridine 5-oxyacetic acid methyl ester, uridine-5-oxyacetic acid, wybutoxosine, pseudouridine, N1-methylpseudouridine, 5-hydroxymethoxycytidine, Q nucleoside, 2-thiocytidine, 5-methyl-2-thiouridine, 2-thiouridine, 4-thiouridine, 5-thiouridine, N-((9-β-D-ribofuranosyl-6-yl)-carbamoyl)threonine, 2'-O-methyladenosine-5-methyluridine, 2'-O-methyladenosine, 2'-O-methylcytidine, Wybutosine, 3-(3-amino-3-carboxypropyl)uridine, N⁶-acetyladenosine, and 2-methylthio-N⁶-methyladenosine; and preferred functional nucleotide analogs include at least one, two, or three of pseudouridine, N1-methylpseudouridine, and 5-hydroxymethoxycytidine.

The present disclosure relates to mRNA containing a functional nucleotide analog.

In some embodiments, the mRNA includes self-replicating mRNA.

In some embodiments, the mRNA includes one or more non-structural replicase domains from an α virus.

In some embodiments, the mRNA includes an integrated target polynucleotide fragment.

The present disclosure relates to a modified self-replicating mRNA, where a polynucleotide fragment of the modified self-replicating mRNA includes one or more non-structural replicase domains from an α virus and an integrated target fragment, where the polynucleotide fragment includes a functional nucleotide analog.

In some embodiments, a proportion of any type of the functional nucleotide analog in the mRNA/self-replicating mRNA is 0.01% to 100%, or may alternatively be 0.1%, 0.5%, 1%, 2%, 3%, 4%, 5%, 6%, 7%, 8%, 9%, 10%, 11%, 12%, 13%, 14%, 15%, 20%, 25%, 30%, 35%, 40%, 50%, 60%, 70%, 80%, or 90%.

In some embodiments, the proportions of all types of the functional nucleotide analogs in the mRNA or self-replicating mRNA are 0.01% to 100%, or may alternatively be 0.1%, 0.5%, 1%, 2%, 3%, 4%, 5%, 6%, 7%, 8%, 9%, 10%, 11%, 12%, 13%, 14%, 15%, 20%, 25%, 30%, 35%, 40%, 50%, 60%, 70%, 80%, or 90%.

The functional nucleotide analog may be located at any position in the mRNA, for example, in either or both of the non-structural replicase domain of the α virus and the integrated target polynucleotide fragment. In some embodiments, a proportion of the functional nucleotide analog located in the non-structural replicase domain of the α virus is 0% to 100%, or, for example, at least 0.01%, 0.1%, 0.5%, 1%, 2%, 3%, 4%, 5%, 10%, 20%, 30%, 40%, 50%, 60%, 70%, 80%, or 90%. In some embodiments, a proportion of the functional nucleotide analog located in the target fragment is 0% to 100%, or, for example, at least 0.01%, 0.1%, 0.5%, 1%, 2%, 3%, 4%, 5%, 10%, 20%, 30%, 40%, 50%, 60%, 70%, 80%, or 90%.

It can be easily understood that the "unmodified form" in the present disclosure refer to the nucleotides of the functional nucleotide analog before modification, and they generally have the same base. For example, the unmodified forms of pseudouridine and N1-methylpseudouridine can be uridine, and the unmodified form of 5-hydroxymethoxycytidine can be cytidine.

In some embodiments, the functional nucleotide analog includes at least one of pseudouridine, N1-methylpseudouridine, 5-hydroxymethoxycytidine, and N⁶-methyladenosine.

In some embodiments, the functional nucleotide analog includes at least one of pseudouridine, N1-methylpseudouridine, and 5-hydroxymethoxycytidine.

In some embodiments, uridine in the unmodified form of the polynucleotide fragment is modified to pseudouridine at a proportion of 1% to 100%, 1% to 50%, 10% to 50%, 20% to 40%, or 25% to 35%; and the modification proportion can also be selected as 5%, 10%, 20%, 25%, 30%, 35%, 40%, 50%, 60%, 70%, 80%, or 90%.

In some embodiments, uridine in the unmodified form of the polynucleotide fragment is modified to N1-methylpseudouridine at a proportion of 1% to 100%, 1% to 60%, 10% to 60%, or 10% to 20%; and the modification proportion can also be selected as 5%, 10%, 15%, 20%, 30%, 40%, 50%, 60%, 70%, 80%, or 90%.

In some embodiments, cytidine in the unmodified form of the polynucleotide fragment is modified to 5-hydroxymethoxycytidine at a proportion of 1% to 100%, 80% to 100%, or 90% to 100%; and the modification proportion can also be selected as 5%, 10%, 20%, 30%, 40%, 50%, 60%, 70%, 80%, or 90%.

In some embodiments, adenosine in the unmodified form of the polynucleotide fragment is modified to N⁶-methyladenosine (m⁶A) at a proportion of 1% to 100%, 1% to 30%, 1% to 10%, or 1% to 5%; and the modification proportion can also be selected as 2%, 3%, 4%, 5%, 6%, 7%, 8%, 9%, 10%, 11%, 12%, 13%, 14%, 15%, 20%, 25%, 30%, 35%, 40%, 50%, 60%, 70%, 80%, or 90%.

In some embodiments, the target fragment includes at least one mRNA encoding an antigen or its fragment or epitope; preferably, the antigen is a pathogenic antigen, and more preferably, a viral antigen, bacterial antigen, parasitic antigen, fungal antigen, protozoan antigen, prion antigen, or tumor antigen.

Optionally, the virus for the viral antigen includes one or more of *adenoviridae, arenaviridae, astroviridae, bunyaviridae, caliciviridae, flaviviridae, hepeviridae, mononegavirales, nidovirales, picornaviridae, orthocoronavirinae, orthomyxoviridae, papillomaviridae, parvoviridae, polyomaviridae, poxviridae, reoviridae, retroviridae,* and *togaviridae.*

Optionally, the bacterium for the bacterial antigen includes one or more of *Staphylococcus, Streptococcus, Listeria, Erysipelothrix, Renibacterium, Bacillus, Clostridium, Mycobacterium, Actinomycetes, Nocardia, Corynebacterium, or Rhodococcus;* and/or one or more of *Bacillus anthracis, Erysipelothrix, Tetanus bacillus, Listeria bacillus, Bacillus carbonis, Mycobacterium tuberculosis, Escherichia coli, Proteus, Dysentery bacillus, Pneumobacillus, Brucella, Clostridium perfringens, Haemophilus influenzae, Haemophilus parainfluenzae, Moraxella catarrhalis, Acinetobacter, Yersinia, Legionella pneumophila, Pertussis bacillus, Bacillus parapertussis, Shigella, Pasteurella, Vibrio cholerae,* and *Vibrio parahaemolyticus.*

Optionally, the fungus for the fungal antigen includes one or more of *Coccidioides immitis, Coccidioides posadasii, Histoplasma capsulatum, Histoplasma duboisii, Loboa loboi, Paracoccidioides brasiliensis, Blastomyces dermatitidis, Sporothrix schenckii, Penicillium marneffei, Candida albicans, Candida glabrata, Candida tropicalis, Candida lusitaniae, Aspergillus, Exophiala jeanselmei, Fonsecaea pedrosoi, Fonsecaea compacta, Phialophora verrucosa, Exophiala dermatitidis, Geotrichum candidum, Pseudallescheria boydii, Cryptococcus neoformans, Trichosporon, Rhizopus oryzae, Mucor indica, Absidia corymbifera, Syncephalastrum racemosum, Basidiobolus ranarum, Conidiobolus coronatus, Conidiobolus incongruus, Rhinosporidium seeberi,* Hyalohyphomycosis fungus, and Phaeohyphomycosis fungus.

Optionally, the parasite includes one or more of a gastrointestinal parasite, a hepatic parasite, a pulmonary parasite, a brain tissue parasite, an intravascular parasite, a lymphatic parasite, a muscle tissue parasite, an intracellular parasite, a bone tissue parasite, and an ocular parasite.

Optionally, the tumor includes a tumor generated in any of the following: bone, synostosis, muscle, lung, trachea, heart, spleen, artery, vein, blood, capillary, lymph node, lymphatic vessel, lymph fluid, oral cavity, pharynx, esophagus, stomach, duodenum, small intestine, colon, rectum, anus, appendix, liver, gallbladder, pancreas, parotid gland, sublingual gland, urinary kidney, ureter, bladder, urethra, ovary, fallopian tube, uterus, vagina, vulva, scrotum, testicle, vas deferens, penis, eye, ear, nose, tongue, skin, brain, brainstem, medulla, spinal cord, cerebrospinal fluid, nerve, thyroid, parathyroid gland, adrenal gland, pituitary gland, pineal gland, pancreatic islet, thymus, gonad, sublingual gland, and parotid gland.

In some embodiments, the target fragment is an mRNA derived from SARS-CoV-2, preferably spike protein mRNA or a fragment thereof, or more preferably, the target fragment includes RBD gene or a fragment thereof.

In some embodiments, the α virus is selected from at least one of TC83 Venezuelan Equine Encephalitis Virus (VEEV), Sin-dbis virus, Chikungunya virus, Eastern equine encephali-tis virus, Western equineencephalitis virus, Mayaro virus, Semliki forest virus, and Venezuelan equine encephalitisvirus. Preferably, the α virus is TC83 Venezuelan Equine Encephalitis Virus (VEEV). However, it should be understood that the α viruses listed herein are only illustrative, but not limited thereto.

In some embodiments, a nucleotide sequence of the unmodified form of the modified self-replicating mRNA is shown as SEQ ID NO: 3.

The present disclosure further relates to a vaccine composition including the modified self-replicating mRNA as described above.

In some embodiments, the vaccine composition further includes at least one of a pharmaceutically acceptable carrier, diluent and excipient.

The term "pharmaceutically acceptable" refers to molecules, molecular fragments, or compositions that, when appropriately administered to animals or humans, do not cause adverse, allergic, or other undesirable reactions. Specific examples of substances that can serve as pharmaceutically acceptable carriers or their components include phosphoric acid, citric acid, and other organic acids; antioxidants (for example, ascorbic acid and methionine); antimicrobial agents (for example, phenyldimethyl octadecyl ammonium chloride, hexamethonium chloride, benzalkonium chloride, phenol, butanol or benzyl alcohol, alkylparaben, catechol, resorcinol, cyclohexanol, 3-pentanol, or m-cresol); low-molecular weight (about less than 10 kDa) polypeptides; proteins such as serum albumin, gelatin, or immunoglobulins; hydrophilic polymers such as polyvinylpyrrolidone; amino acids (for example, glycine, glutamine, asparagine, histidine, arginine, or lysine); monosaccharides, disaccharides, and other carbohydrates (including, for example, glucose, mannose, or dextran); chelating agents (for example, EDTA); sugars (for example, sucrose, mannitol, trehalose, or sorbitol); salt counterions; metal composites; and/or non-ionic surfactants (including, for example, TWEEN^{™}, PLURONICS^{™}, or polyethylene glycol). In addition, depending on the formulation method, common fillers, diluents, binders, moisturizers, disintegrants, and/or surfactants can be suitably selected by those skilled in the art. The vaccine composition can be present in solid, semi-solid, or liquid form, preferably in liquid form.

In some embodiments, the vaccine composition also includes a nucleic acid stabilizer.

Examples of stabilizers for stabilizing and maintaining nucleic acids include cationic compounds, detergents, chaotropic salts, ribonuclease inhibitors, chelating agents, and mixtures thereof, among others. The stabilizers can include, for example, crosslinking fixatives such as paraformaldehyde or precipitants such as ethanol. The stabilizers can function by forming covalent bonds between cell molecules, precipitating some intracellular molecules, or by other means. In some embodiments, the stabilizers include cell lysis buffers. Cell permeabilization buffers are also known in the art and can contain detergents that permeabilize a cell membrane to allow probes and dyes to pass through the membrane. Examples of detergents used in cell lysis buffers include, but are not limited to, Tween, Triton X-100, saponin, and NP-40. The concentrations of cell lysis buffers and permeabilizers are adjusted for the final intended purpose. Optimal effects may not be achieved when the cell lysis buffers and permeabilizers are at excessively low concentrations. When at excessively high concentrations, undesirable cell damage may occur. Routine and empirical steps can be carried out to determine the preferred concentrations in each case. In some embodiments, the stabilizers include chloroform, phenol, and TRIZOL. However, in a more preferred embodiment, the stabilizer is a component that is easy to remove or has low cytotoxicity, and most preferably, is a pharmaceutically acceptable component.

The vaccine provided in the present disclosure preferably also includes an adjuvant. Adjuvants suitable for the vaccines of the present disclosure include those that can enhance the immunogenicity of self-replicating mRNA, particularly the target fragments thereof. For example, adjuvants for the antibody response to B-cell epitopes of the antigen, as well as adjuvants that can enhance cell-mediated responses to T-cell epitopes in the antigen. These adjuvants are well known in the field.

In some embodiments, the adjuvant is selected from one or more of alum, complete Freund's adjuvant, incomplete Freund's adjuvant, squalene, squalane, muramyl dipeptide, MF59, AS03, monophosphoryl lipid A, flagellin, CpG-ODN, Poly(I:C), and small molecules of aluminum or calcium salts. These adjuvants are all well known in the art and commercially available.

Complete Freund's adjuvant, incomplete Freund's adjuvant, squalane, and alum are generally not used in humans.

In some embodiments, the vaccine is a water-in-oil emulsion with an aqueous phase and an oil phase.

In some embodiments, the vaccine is an oil-in-water emulsion with an aqueous phase and an oil phase.

The vaccine is typically prepared for parenteral administration. Typical immunizations are implemented via oral and subcutaneous (SC) routes, nasal route, or intramuscular (IM), intravenous (IV), intraperitoneal (IP), or intradermal (ID) injection.

The vaccine is administered appropriately to the dosage and formulation, such as therapeutic and immunogenic effective dosages. The dosage depends on the subject under treatment, the antibody synthesization ability of the subject's immune system, and the expected degree of protection. The exact amount of the active component to be administered is at the discretion of a physician and varies from individual to individual. Appropriate treatments for initial administration and booster vaccination can also vary, but typically, the first administration is followed by one injection or administration in another method after a certain period (several weeks or months).

In some embodiments, the vaccine composition is packaged and delivered in the form of a plasmid, a viral vector, a liposome, a dendritic macromolecule, an inorganic nanoparticle, or a cell-penetrating peptide.

The mRNA molecule can be packaged directly or as a precursor of the mRNA molecule. Plasmids and viral vectors may contain a selective marker (for example, a tag that facilitates enrichment, such as a his tag; or a tag that is easy to detect, such as GFP), and a replication origin that matches the cell type specified by the cloning vector, where the cloning vector includes regulatory elements required to affect expression in a specified target cell. The viral vectors can be bacteriophages, lentiviruses, retroviruses, adenoviruses, or adeno-associated viruses.

The liposomes can be cationic or neutral liposomes, and can be prepared or modified using well-known methods. For example, adding polyethylene glycol (PEG)-modified liposomes can effectively prevent aggregation of liposome vectors and increase their stability.

Dendritic macromolecules are a special family of polymers with definite molecular structures whose chemical structures and unique multivalent characteristics can be precisely controlled, and have gradually become non-viral vectors for gene transfer. Typical dendritic macromolecules, such as poly(amidoamine) (PAMAM) dendritic polymers, can be further modified. For example, the surface of PAMAM can be modified with a nucleobase analog 2-amino-6-chloropurine to construct a derivative, AP-PAMAM, or chondroitin sulfate (CS) can be conjugated with PAMAM to prepare CS-PAMAM, and so on.

The inorganic nanoparticles can be selected as gold nanoparticles (AuNPs), magnetic nanoparticles, mesoporous silica nanoparticles (MSNs), and so on.

The cell-penetrating peptides (CPPs) are a class of small molecule peptides with strong transmembrane transport ability, and can transport various macromolecular substances such as polypeptides, proteins, and nucleic acids into cells. CPPs can be categorized into cationic CPPs (for example, TAT, Penetratin, Polyarginine, P22N, DPV3, and DPV6), amphiphilic CPPs (which can be created by linking hydrophobic peptide sequences to NLSs covalently or by isolating from natural proteins such as pVEC, ARF (1-22), and BPrPr (1-28)), and hydrophobic CPPs (generally containing only non-polar amino acid residues and having net charges about 20% less than the total charge of the amino acid sequence).

Another embodiment of the present disclosure relates to a kit, where the kit includes the vaccine as described above and a container for administering the vaccine composition.

The container for inoculation is preferably a medical syringe.

The present disclosure further relates to a method for modifying the following properties of mRNA after inoculation into an animal:
a) improvement of immunogenicity of the mRNA; and/or
b) improvement of the mRNA's ability to evade immune surveillance;
where the method includes substituting an unmodified nucleotide in the mRNA with a corresponding functional nucleotide analog.

In some embodiments, the mRNA is self-replicating mRNA, preferably, the above-described modified self-replicating mRNA obtained after substitution.

In some embodiments, the animal can be a chicken, duck, goose, cat, dog, cattle, sheep, horse, donkey, pig, giant panda, monkey, rabbit, rat, or human.

The embodiments of the present disclosure are described in detail below with reference to examples. It should be understood that these examples are only used to illustrate the present disclosure and are not intended to limit its scope. In the following examples, for a test method where a specific condition is not specified, preferably, refer to the guidance provided in the present disclosure, a test manual, conventional conditions in the art, another known test method in the art, or a condition recommended by a manufacturer.

In the following specific examples, a measurement parameter related to an ingredient may have a slight deviation within a weighing accuracy range unless otherwise specified. For temperature and time parameters, acceptable deviations due to instrument test accuracy or operation accuracy are allowed..

### Example 1

### 1. Synthesis of RBD Gene Fragment

A spike protein (SARS-CoV-2) gene was retrieved from NCBI, and the receptor binding domain (RBD) gene was redesigned and optimized according to human codon usage. The nucleotide sequence is denoted as SEQ ID NO: 1. An ApaI cleavage site and a promoter were added upstream, and a NotI cleavage site was added downstream. Finally, the DNA sequence was directly synthesized (obtained in the form of a cloning plasmid pUC57-RBD from a company).

### 2. Construction of TC83 Self-Replicating Vector

The self-replicating mRNA was designed according to the genome of TC83 Venezuelan Equine Encephalitis Virus (TC83, VEEV) in the α virus family, and contained genes encoding α virus self-replicating components but lacked structural proteins necessary for infectious α virus particles. The designed sequence was directly synthesized, and its nucleotide sequence is shown as SEQ ID NO: 2.

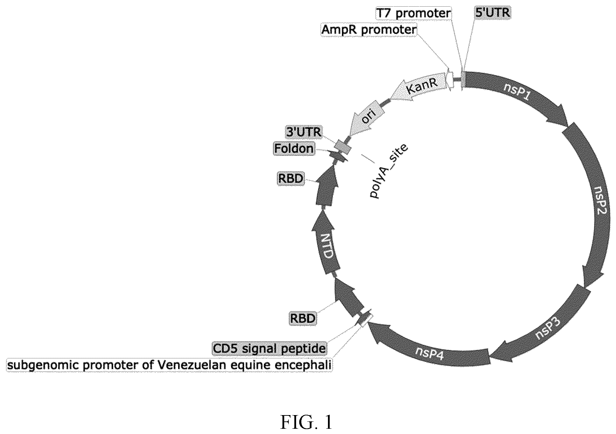

### 3. Preparation Method of Recombinant Plasmid JCXH-107 (the prepared recombinant plasmid JCXH-107 is shown in FIG. 1)

The pUC57-RBD and TC83 self-replicating vector were subjected to double enzyme digestion with ApaI and NotI, in an enzyme digestion system of 20 µL: <1 µg of the pUC57-RBD or TC83 self-replicating vector, 1 µL of ApaI, 1 µL of NotI, 2 µL of a 10×CutSmart buffer, and ddH₂O to make up to 20 µL. The plasmid was digested in a water bath at 25°C for 1 hour. Then, the digestion continued in a water bath at 37°C for 1 hour. To dephosphorylate the vector fragments, 0.5 µL of CIP was added, and the mixture was incubated in a water bath at 37°C for 30 minutes. The digested mixture was mixed with a 6× loading buffer and subjected to electrophoresis (1% agarose, 94V). Fragments of corresponding lengths were obtained by gel extraction (the pUC57-RBD fragment was 2574 bp; the TC83 self-replicating vector fragment was about 9.5 Kb), and eluted with 30 µL of elution buffer.

The RBD gene fragment and TC83 self-replicating vector fragment were taken according to the following ligation system: 50 ng of the vector, the molar ratio of the inserted fragment to the vector fragment = 5:1, 1 µL of T4 ligase, and 5 µL of a 10× ligase buffer, ddH₂O to make up to 10 µL, and the mixture was subjected to ligation at 22°C for 1 hour. The ligation product was gently mixed with competent cells of Escherichia coli DH5α at a 1:10 volume ratio, subjected to ice bath for 30 minutes, thermal shock at 42°C for 45 seconds, and ice bath for 3 minutes. 500 µL of preheated SOB broth was added, mixed uniformly, then cultured at 37°C and 180 rpm for 1 hour, plated on an LK plate (LB-Kan plate: LB plate containing 50 µg/mL of Kan) and cultured at 37°C for 16-20 hours.

PCR Screening: By using a bacterial plasmid extracted by boiling as a template, the spike protein of the target band was amplified with an upstream primer F: 5'-TATGGCCATGACTACTCTAGCTA-3' and a downstream primer R: 5'-GGGAAACGCCTGGTATCTTT-3'. The reaction cycle conditions were: 94°C for 3 minutes→ (94°C for 1 minute, 47°C for 30 seconds, and 72°C for 3 minutes) × 30 cycles→ 72°C for 10 minutes→ 4°C. The PCR result was observed through electrophoresis (electrophoresis conditions: 1% agarose gel; 90V, loading volume: 5 µL of the PCR product), and the expected result was that the RBD fragment was about 2977 bps.

Enzyme Digestion Verification: Plasmids were extracted from positive colonies verified by PCR screening, digested with ApaI and NotI according to the aforementioned enzyme digestion system, and the digested mixture was mixed with a 6× loading buffer for electrophoresis (1% agarose, 94V).

Sequencing Verification: Plasmids verified by PCR and enzyme digestion were sent to a sequencing company for sequencing. The *E. coli* carrying the positive plasmid verified to be completely correct by sequencing was stored at -80°C.

### 4. Method for Linearizing and Recovering JCXH-107 Plasmid Through Enzyme Digestion With BspQI

10 µg of the JCXH-107 plasmid, 1 µL of BspQI, 3.15 µL of 10×NE buffer, and ddH₂O to make up to 50 µL were added to the system, and the mixture was subjected to a water bath at 50°C for 1 hour to digest the plasmids with the enzyme. The digested mixture was mixed with 6× loading buffer and subjected to electrophoresis (1% agarose, 94V), and the fragments with the corresponding length (for the JCXH-107 plasmid, about 12 Kb) were recovered from the gel and eluted with 30 µL of elution buffer.

5. The *in vitro* transcription reaction (IVT) of the linearized JCXH-107 plasmid was initiated using T7 RNA polymerase. Turbo DNase enzyme was used to degrade the template DNA, and capping enzyme (Vaccinia capping enzyme) was used to add a 7-methylguanylate cap structure (referred to as Cap 0) to the 5' end of the transcribed mRNA. The specific method was as follows:

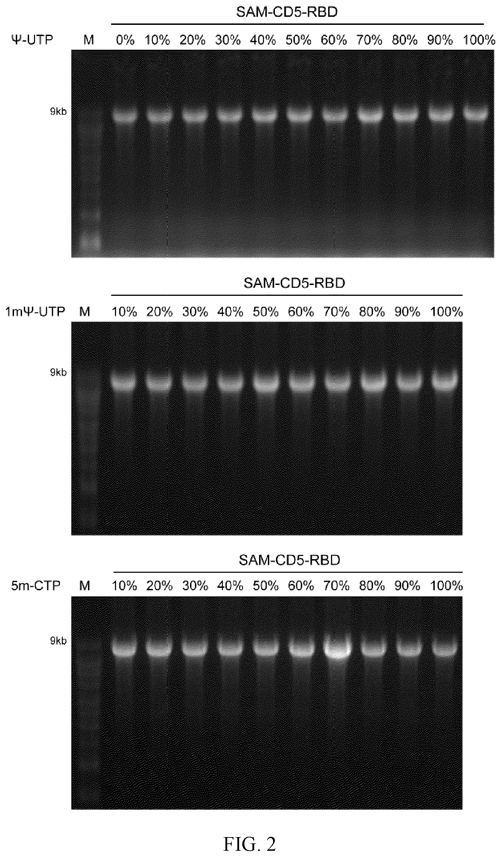

2 µL of 10× reaction buffer and 0.5 mM of each type of NTP were added. Uridine (U) may be replaced with pseudouridine in a proportion of 10%, 20%, 30%, 40%, 50%, 60%, 70%, 80%, 90%, or 100%, or uridine (U) may be replaced with N1-methylpseudouridine in a proportion of 10%, 20%, 30%, 40%, 50%, 60%, 70%, 80%, 90%, or 100%; cytidine (C) may be replaced with 5-hydroxymethoxycytidine in a proportion of 10%, 20%, 30%, 40%, 50%, 60%, 70%, 80%, 90%, or 100%; 1 µg of the linearized JCXH-107 and 2 µL of T7 RNA Polymerase were added, and water was added to make up to 20 µL. The mixture reacted at 30°C for 1 hour, then 1 µL of TURBO^{™} DNase, 4 µL of 10× capping buffer, 2 µL of GTP (10mM), 2 µL of SAM (2mM), and 2 µL of vaccinia capping enzyme were added, and water was added to make up to 40 µL. The mixture reacted at 30°C for 1 hour. Then water was added to make up to 200 µL, followed by 120 µL of 7.5M lithium chloride, mixed uniformly, allowed to stand at -20°C for 30 minutes, and centrifuged at 14,000 g and 4°C for 30 minutes. The supernatant was discarded, and the precipitate was washed with 70% alcohol, centrifuged at 14,000 g and 4°C for 5 minutes. The supernatant was discarded, and the precipitate was dried in the air for 5 minutes. The precipitate was then dissolved in 40 µL of water. After quantification by spectrophotometry, 400 ng of the solution was taken and mixed with 10 µL of Northern Max-Gly Sample Loading Dye, incubated at 50°C for 30 minutes, and subsequently subjected to electrophoresis with Northern Max-Gly Gel Prep/Running buffer (1% agarose, 70 V). Electrophoresis results of the self-replicating mRNA-RBD (denoted as SAM-CD5-RBD in the figures) after *in vitro* transcription are shown in FIG. 2, and the nucleotide sequence in an unmodified form is shown as SEQ ID NO:3.

6. Western Blot Detection of Expression of Self-Replicating mRNA-RBD in Cells. BHK cells purchased from the Shanghai Cell Center were subcultured. When the number of cells was sufficient, the cells were digested with pancreatin, placed into each well of a 6-well plate, and incubated in a CO₂ incubator at 37°C overnight. On the next day, the liposome-coated mRNA-RBD was used to transfect the plated BHK cells and cultured for 24 hours. Then the cells were lysed to collect a protein sample. The specific method was as follows:
1) Cell Culture and Incubation: Thawed BHK cells were inoculated into a 75 cm² culture flask with a medium of DMEM high-glucose medium + 5% double antibody + 10% fetal bovine serum. When the density of the cells at the bottom of the flask reached above 80%, the cells were digested with pancreatin and counted. A suitable number of cells were incubated in a 6-well cell culture plate and placed in a CO₂ incubator at 37°C overnight.
2) Transfection of BHK or Hela cells with Liposome mRNA: The medium in the 6-well plate was removed, and the wells were washed once with PBS buffer. 0.5µg of the liposome-coated mRNA was mixed with 200 µL of Opti-MEM medium and added to the washed 6-well plate. The cells were incubated in a CO₂ incubator at 37°C for 6 hours, then supplemented with 200 µL of DMEM high-glucose medium containing 20% fetal bovine serum. The cells were further incubated in the CO₂ incubator at 37°C for 18 hours.
3) Treatment of Protein Sample: 24 hours later, 200 µL of cell lysis solution and 1% PMSF were added to the BHK cells, placed on ice for 5 minutes, and centrifuged at 14000 g for 5 minutes. The supernatant was transferred to a new centrifuge tube, 5×SDS was added, and the mixture was incubated in a metal bath at 95°C for 12 minutes. The solution was stored at -20°C for later use.

### 4) Western Blot Detection:

Electrophoresis: A polyacrylamide separation gel with a concentration of 6% was used. The protein loading volume was 20 µL. Spacer gel electrophoresis conditions were 150V for 10 minutes, and separation gel electrophoresis conditions were 200V for 30 minutes.

Electroblotting: Membrane transfer was conducted using a nitrocellulose membrane at a voltage of 100V for 1 hour.

Blocking: 5% BSA prepared with 1×PBST was used as a blocking solution, and the mixture was subjected to blocking at 4°C overnight.

Incubation of Primary Antibody: The SARS-CoV-2 (2019-nCov) spike protein antibody was diluted at 1:2000 with the blocking solution and incubated at room temperature for 1 hour. The membrane was washed with 1×PBST 3 times, each wash lasting 10 minutes.

Incubation of Secondary Antibody: The secondary antibody was diluted with the blocking solution at a dilution ratio of 1:5000 and incubated at room temperature for 1 hour. The membrane was washed with 1×PBST 3 times, each wash lasting 10 minutes.

Development: Development was conducted in an imaging system with developing solution A and B in a 1:1 ratio.

The experimental results are shown in FIG. 3-1 and FIG. 3-2. FIG. 3-1 and FIG. 3-2 compare the expression of RBD proteins after BHK cells were transfected *in vitro* with self-replicating mRNA-RBD (denoted as SAM-CD5-RBD in the figures) with modified nucleotides and conventional nucleotides, showing expression detection of RBD proteins in cell lysates and cell supernatants. Actin protein was used as the internal reference in the cell lysate, and proteins subjected to Coomassie Blue Staining were used as the internal reference in the cell supernatant. FIG. 4 is a statistical diagram of RBD proteins in cell lysates from FIG. 3-1 and FIG. 3-2. It can be seen from FIG. 3-1, FIG. 3-2, and FIG. 4 that the expression of RBD proteins after BHK cells were transfected *in vitro* with self-replicating mRNA-RBD was good.

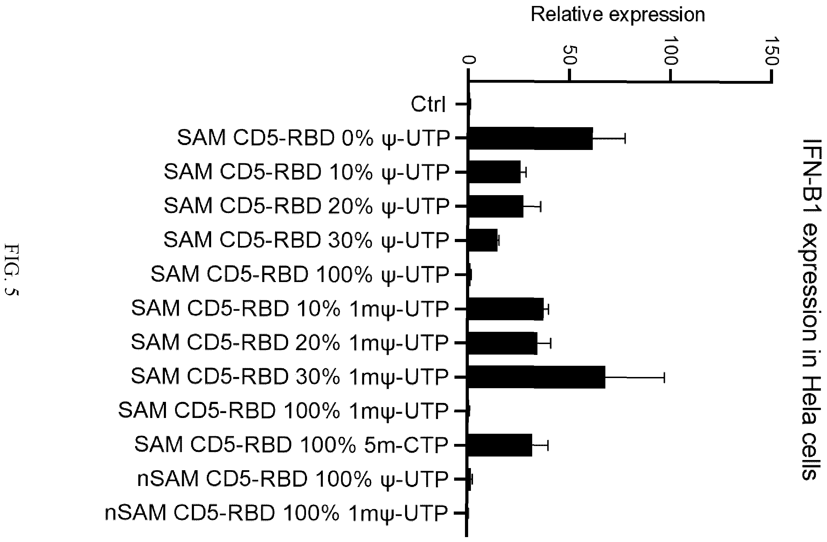

7. The present disclosure compared the transcription levels of the natural immune regulatory factor IFN-B1 by real-time fluorescence quantitative PCR after transfection of Hela cells *in vitro* with mRNA-RBD obtained from *in vitro* transcription reactions of self-replicating mRNA-RBD (denoted as SAM-CD5-RBD in the figures) with modified nucleotides and conventional nucleotides. The specific method was as follows:
1) Extraction of RNA from Cells: The medium of the cells was pipetted out, the cells were washed with PBS once, 1 mL of RNAiso was added, the cells were subjected to blowing and stirring and collected into an EP tube. 0.2 mL of chloroform was added, the mixture was mixed under shaking, allowed to stand for 5 minutes, and centrifuged at 12,000 g for 15 minutes. The supernatant was pipetted. Isopropanol of the same volume as the supernatant was added, and the mixture was mixed under shaking and allowed to stand at room temperature for 10 minutes. The mixture was centrifuged at 12,000 g for 10 minutes, and the supernatant was discarded. The precipitate was washed by adding 1 mL of nuclease-free water containing 75% ethanol, centrifuged at 12,000 g for 5 minutes, and the supernatant was discarded. The precipitate was air-dried, 20 µL of nuclease-free water was added, and the precipitate was subjected to blowing and stirring gently to dissolve it. The OD260/OD280 value and RNA concentration were determined using an ultraviolet spectrophotometer.
2) Preparation of cDNA Samples: 1.5 µg of RNA samples were taken, 5× gDNA Digester Mix and nuclease-free water were added for dilution to a final volume of 15 µL, and the mixture was incubated at 42°C for 2 minutes. 2 µL of 10× Hifair^{®} III Super Buffer, 1 µL of Hifair^{®} III RT Enzyme Mix, 1 µL of Random Primers N6, and 1 µL of nuclease-free water were added, mixed uniformly, and subjected to a reverse transcription reaction. The reverse transcription procedures were carried out at 25°C for 5 minutes, at 60°C for 15 minutes, and at 85°C for 5 minutes.
3) Detection by Real-time Fluorescence Quantitative PCR: The cDNA sample was diluted 5-fold, and the following reaction system was formulated: 5 µL of 2× ChamQ Universal SYBR qPCR Master Mix, 0.2 µL of 10 µM upstream primer, 0.2 µL of 10 µM downstream primer, 1 µL of diluted cDNA sample, and 3.6 µL of nuclease-free water. The mixture was mixed uniformly and used for detection. For detecting the expression of the IFN-B1 gene, the sequence of the upstream primer was CATTACCTGAAGGCCAAGGA, and the sequence of the downstream primer was CAGCATCTGCTGGTTGAAGA. For detecting the expression of the internal reference GAPDH gene, the sequence of the upstream primer was GAAGGCTGGGGCTCATTT, and the sequence of the downstream primer was CAGGAGGCATTGCTGATGAT. For detecting the expression of the RIG-I gene, the sequence of the upstream primer was GTTGTCCCCATGCTGTTCTT, and the sequence of the downstream primer was GCAAGTCTTACATGGCAGCA. Detection was conducted using a Thermo Fisher Quant Studio 1 real-time fluorescence quantitative PCR system. The detection procedure was: stage 1: 95°C for 3 minutes, repeated once; stage 2: 95°C for 10 seconds, and 60°C for 30 seconds, repeated 40 times; and stage 3: melting curve: 95°C for 15 seconds, 60°C for 60 seconds, and 95°C for 15 seconds, repeated once. The expression of the target gene was analyzed using a relative quantification method: fold change = 2^{-ΔΔCT}. The results are shown in FIG. 5. As a positive control, non-self-replicating mRNA-RBD with 100% pseudouridine modification and non-self-replicating mRNA-RBD with 100% N1-methylpseudouridine modification almost did not stimulate high expression of the natural immunomodulatory factor IFN-B 1 gene after cell transfection. However, self-replicating mRNA-RBD with conventional nucleotide modification stimulated high expression of IFN-B1 after cell transfection. Compared with the self-replicating mRNA-RBD with conventional nucleotide modification, self-replicating mRNA-RBD with 10%, 20%, and 30% pseudouridine modifications lowered the stimulation of IFN-B 1 after cell transfection, and as the proportion of modified nucleotide increased, the decrease in IFN-B 1 was more significant, with little impact on the translation of the RBD protein. Self-replicating mRNA-RBD with 10%, 20%, and 30% N1-methylpseudouridine modifications also lowered high expression of IFN-B1 after cell transfection, with little impact on the translation of the RBD protein. However, self-replicating mRNA-RBD with 100% pseudouridine modification and self-replicating mRNA-RBD with 100% N1-methylpseudouridine modification almost did not stimulate high expression of the IFN-B1 gene after cell transfection, but significantly inhibited the translation of the RBD protein. Self-replicating mRNA-RBD with 100% 5-methylcytidine modification lowered high expression of IFN-B1 to a certain extent after cell transfection, but not as effectively as self-replicating mRNA-RBD with 30% pseudouridine modification, with little impact on the translation of the RBD protein. Through comprehensive comparison of the effects of different types and degrees of nucleotide modifications on the expression of the IFN-B1 gene and the translation of the RBD protein, self-replicating mRNA-RBD with 30% pseudouridine modification showed better inflammatory gene inhibition effects than self-replicating mRNA-RBD with conventional nucleotide modification, while retaining good translation of the RBD protein.

### 8. Step of Packaging mRNA-RBD with Lipids.

mRNA and lipids (including 1,2-dimyristoyl-racemic-glycerol-3-methoxypolyethylene glycol-2000 (DMG-PEG2000), cholesterol, distearoyl phosphatidylcholine (DSPC), and cationic lipids, which were dissolved in alcohol) were quickly mixed using a Nanoassemblr mixer, causing the precipitation of the lipids and the encapsulation of the mRNA into the LNP due to electrostatic interactions. The mRNA-RBD-LNP complex was then concentrated and exchanged into a preparation solution.

### 9. Detection of Titer of Anti-Spike Protein Antibody Induced in Mice Immunized with mRNA-RBD-LNP Vaccine

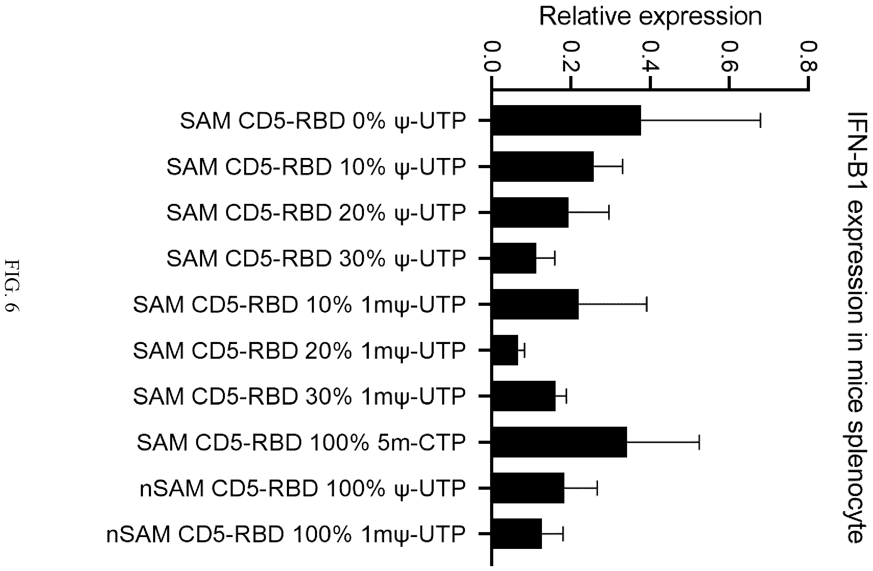

6- to 8-week-old BALB/c female mice were divided into groups after 3 days of adaptive feeding, with 6 mice in each group. One group was used as the control group and injected with a buffer solution, while the other ten groups were injected respectively with self-replicating mRNA-RBD-LNP with conventional nucleotides, self-replicating mRNA-RBD-LNP with 10%, 20%, and 30% pseudouridine modifications, self-replicating mRNA-RBD-LNP with 10%, 20%, and 30% N1-methylpseudouridine modifications, self-replicating mRNA-RBD-LNP with 100% 5-methylcytidine modification, non-self-replicating mRNA-RBD-LNP with 100% pseudouridine modification, and non-self-replicating mRNA-RBD-LNP with 100% N1-methylpseudouridine modification. Each mouse was injected with 3 µg of mRNA-RBD-LNP in the spine on day 0, and spleens of three mice in each group were removed on day 2 to detect IFN-B 1 expression. The results are shown in FIG. 6. For the remaining three mice in each group, after serum collection on days 7 and 14, each mouse was injected with a second dose of 3 µg mRNA-RBD-LNP in the spine, and then mouse serum was collected on days 21 and 28 to detect titers of antibodies against RBD via ELISA. The experimental procedure and design are shown in FIG. 7.

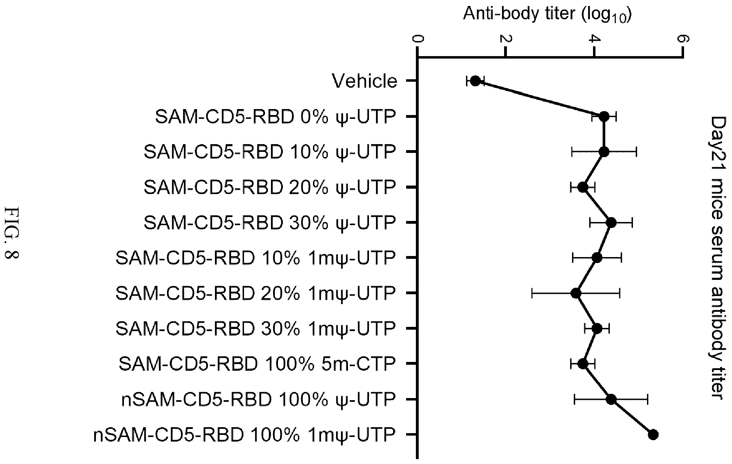

The antibody titer detection method was ELISA, and the specific procedure was as follows: A 96-well ELISA plate was coated with 50 ng of the spike protein (RBD domain protein) per well at room temperature overnight. The next day, the plate was washed with PBST 3 times, blocked with 5% milk, incubated at 37°C for 1 hour, and washed with PBST 3 times. Mouse serum was added at the corresponding dilution ratio, incubated at 37°C for 1 hour, and washed with PBST 3 times. Anti-mouse IgG heavy and light chain HRPs were added, incubated at 37°C for 1 hour, and washed with PBST 3 times, and finally, the developing solution was added for color development. The experiment results are shown in FIG. 8. Mice injected with non-self-replicating mRNA-RBD-LNP with 100% pseudouridine modification and non-self-replicating mRNA-RBD-LNP with 100% N1-methylpseudouridine modification had higher RBD-specific antibodies in their serums. For the self-replicating mRNA-RBD-LNP, mice injected with the self-replicating mRNA-RBD-LNP with 30% pseudouridine modification had higher RBD-specific antibodies in their serums.

From the results of the above examples, it can be seen that the self-replicating mRNA obtained by nucleotide modification using the method according to the present disclosure can be well expressed in cells and can induce a good antibody response in animals.

### Example 2

On the basis of Example 1, the modification type was changed to m⁶A. A brief description is as follows:

### I. Experimental Method

### 1. Cell-level Detection

1.1. The SrRNA-TC83 self-replicating mRNA system was used in this experiment, and the inserted fragment was RBD.
*1.2. In vitro* Synthesis of m⁶A-modified Self-replicating mRNA: Self-replicating mRNAs with different m⁶A modification proportions of 0%, 1%, 5%, 10%, 20%, 30%, 40%, 50%, 60%, 70%, 80%, 90%, and 100% were designed and synthesized.
1.3. Cell Transfection: In this experiment, BHK and Hela cell lines were used for transfection, and Lipofectamine lipid transfection reagent was used at a transfection dosage of 0.5 µg. Samples were collected after 24 hours.
1.4. Result Detection: The changes in the expression levels of RBD proteins in BHK and Hela cells and the changes in the expression of IFN-B1 and/or RIG-I genes in Hela cells were detected by WB.

### 2. Animal Experiment:

2.1. LNP was used to encapsulate self-replicating mRNAs with different m⁶A modification proportions for the animal experiment.
2.2. SrRNA-TC83 was used as a vector to detect protein expression of hEPO (human Erythropoietin) and Luciferase genes in the animal experiment.
2.3. LNP-hEPO-mRNA was administered via intravenous injection in the animal experiment, and LNP-Luciferase-mRNA was administered via intramuscular injection in the animal experiment.
2.4. Protein expression of hEPO *in vivo* was detected by WB, and protein expression of Luciferase was detected by fluorescence imaging *in vivo.*

### II. Experimental Results

### 1. Cell-level Experiment

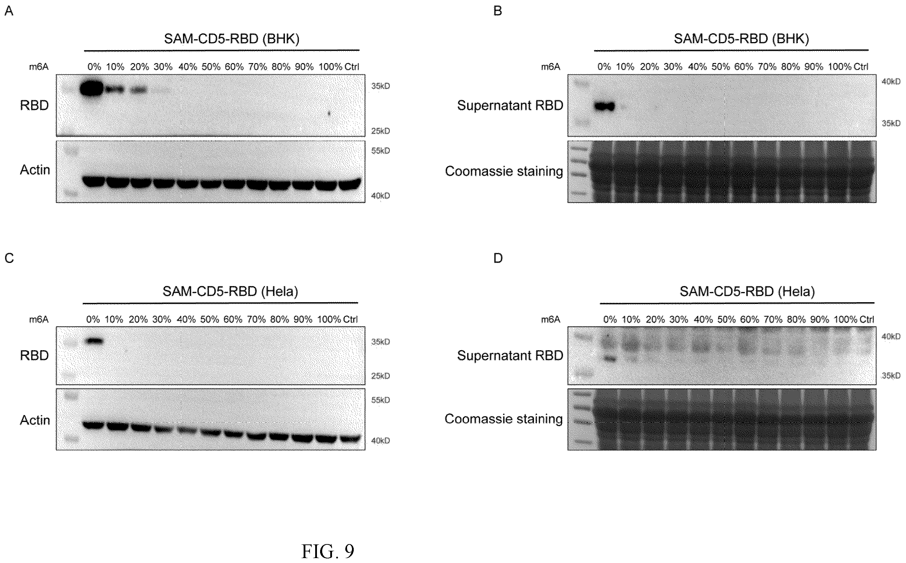

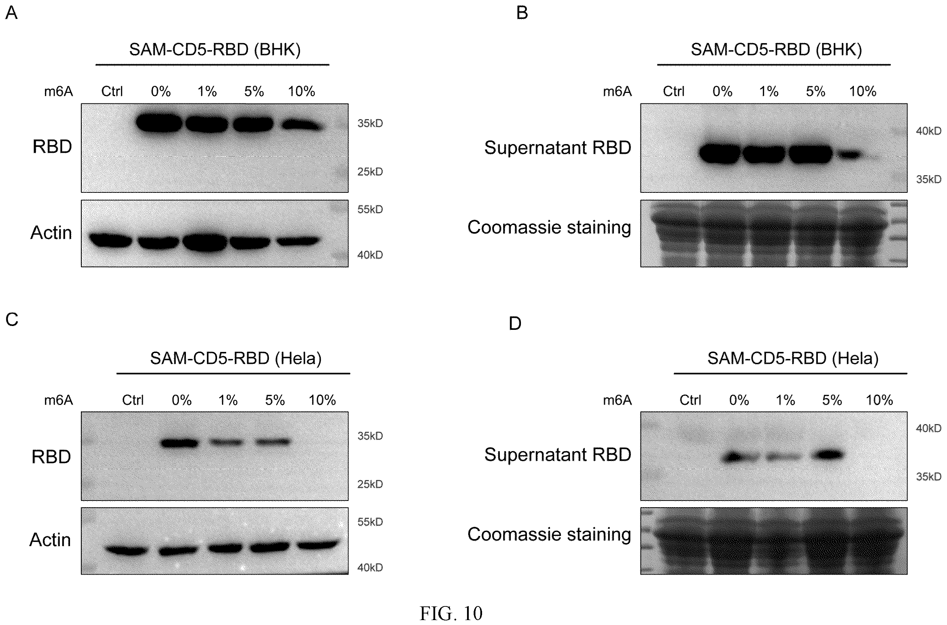

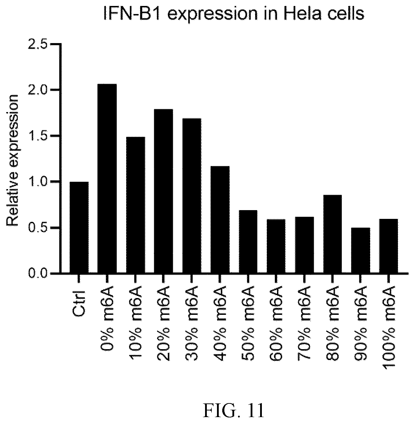

1.1. The expression of RBD protein was not significantly affected or was significantly increased under specific ratios of m⁶A modification (e.g., below 20%, or below 10%, or below 5%), as shown in Figures 9 and 10. - Figure 9 shows protein expression in cell lysates (A and C) and supernatants (B and D). It can be seen that m⁶A modification below 10% did not significantly reduce RBD expression. - WB detection of RBD expression is shown in Figure 10, indicating that 1% and 5% m⁶A modifications did not disrupt RBD expression.
1.2. The expression results of the IFN-B1 gene are shown in FIG. 11, and it can be seen that m⁶A modification can effectively reduce IFN-B1 expression.

### 2. Animal Experiment

2.1. The luciferase fluorescence signal was enhanced with a specific proportion of m⁶A modification.
2.2. The protein expression of hEPO was increased with a specific proportion of m⁶A modification, and the expression of inflammatory genes was significantly decreased in mice.

From the above, it can be seen that m⁶A-modified self-replicating mRNA does not affect protein expression or even promotes protein expression, and can effectively reduces inflammatory gene expression.

Only several examples of the present disclosure are described in the foregoing embodiments. The descriptions are relatively detailed and specific but cannot be construed as limitations on the patent scope of the present disclosure. It should be noted that persons of ordinary skill in the art could also make various variations and improvements without departing from the concept of the present disclosure. These variations and improvements all fall within the protection scope of the present disclosure. Therefore, the scope of the present disclosure should be defined by the appended claims, and the description and drawings may be used to interpret the content of the claims.

## Claims

1. A modified self-replicating mRNA, wherein a polynucleotide fragment of the modified self-replicating mRNA comprises one or more non-structural replicase domains from an α virus and an integrated target fragment, wherein the polynucleotide fragment comprises a functional nucleotide analog; and
wherein the functional nucleotide analog comprises at least one of pseudouridine, N1-methylpseudouridine, 5-hydroxymethoxycytidine, and N⁶-methyladenosine.

2. The modified self-replicating mRNA according to claim 1, wherein uridine in an unmodified form of the polynucleotide fragment is modified to pseudouridine at a proportion of 1% to 100%, 1% to 50%, 10% to 50%, 20% to 40%, or 25% to 35%.

3. The modified self-replicating mRNA according to claim 1, wherein uridine in an unmodified form of the polynucleotide fragment is modified to N1-methylpseudouridine at a proportion of 1% to 100%, 1% to 60%, 10% to 60%, or 10% to 20%.

4. The modified self-replicating mRNA according to claim 1, wherein cytidine in an unmodified form of the polynucleotide fragment is modified to 5-hydroxymethoxycytidine at a proportion of 1% to 100%, 80% to 100%, or 90% to 100%.

5. The modified self-replicating mRNA according to claim 1, wherein adenosine in an unmodified form of the polynucleotide fragment is modified to N⁶-methyladenosine at a proportion of 1% to 100% or 1% to 10%.

6. The modified self-replicating mRNA according to any one of claims 1 to 5, wherein the target fragment comprises at least one mRNA encoding an antigen or a fragment or epitope thereof; preferably, the antigen is a pathogenic antigen, more preferably, the antigen is a viral antigen, bacterial antigen, parasitic antigen, fungal antigen, protozoan antigen, prion antigen, or tumor antigen.

7. The modified self-replicating mRNA according to claim 6, wherein the target fragment is an mRNA derived from SARS-CoV-2, preferably spike protein mRNA or a fragment thereof, more preferably, the target fragment comprises RBD gene or a fragment thereof.

8. The modified self-replicating mRNA according to any one of claims 1 to 5 and 7, wherein the α virus is selected from at least one of TC83 Venezuelan Equine Encephalitis Virus (VEEV), Sin-dbis virus, Chikungunya virus, Eastern equine encephali-tis virus, Western equineencephalitis virus, Mayaro virus, Semliki forest virus, and Venezuelan equine encephalitisvirus.

9. The modified self-replicating mRNA according to claim 8, wherein a nucleotide sequence of an unmodified form of the modified self-replicating mRNA is shown as SEQ ID NO: 3.

10. A vaccine composition comprising the modified self-replicating mRNA according to any one of claims 1 to 9.

11. The vaccine composition according to claim 10, further comprising at least one of a pharmaceutically acceptable carrier, diluent and excipient.

12. The vaccine composition according to claim 10, further comprising a nucleic acid stabilizer and/or an immunoadjuvant.

13. The vaccine composition according to any one of claims 10 to 12, packaged and delivered in a form of a plasmid, a viral vector, a liposome, a dendritic macromolecule, an inorganic nanoparticle, or a cell-penetrating peptide.

14. A kit comprising the vaccine composition according to any one of claims 10 to 12, and optionally a container for administering the vaccine composition.

15. A method for modifying the following properties of mRNA after inoculation into an animal:
a) improvement of immunogenicity of the mRNA; and/or
b) improvement of the mRNA's ability to evade immune surveillance,
wherein the method comprises substituting an unmodified nucleotide in the mRNA with a corresponding functional nucleotide analog.

16. The method according to claim 15, wherein the mRNAis self-replicating mRNA, preferably the modified self-replicating mRNA obtained after substitution according to any one of claims 1 to 9.

17. The method according to claim 15 or 16, wherein the animal is a chicken, a duck, a goose, a cat, a dog, cattle, a sheep, a horse, a donkey, a pig, a giant panda, a monkey, a rabbit, a rat or a human.
